# EUROPEAN PATENT SPECIFICATION

(11) **EP 1 337 293 B1**
(45) Date of publication and mention of the grant of the patent: **08.11.2006**
(21) Application number: 00983866.5
(22) Date of filing: 01.12.2000
(51) Int. Cl.: A61M 25/01

(54) **COMPOSITE GUIDEWIRE**
FÜHRUNGSDRAHT AUS VERBUNDSTOFF
FIL-GUIDE COMPOSITE

(43) Date of publication of application: 27.08.2003
(73) Proprietor: Micrus Corporation, Mountain View, California 94043 (US)
(72) Inventor: FERRERA, David, A., Manhattan Beach, CA 90266 (US)
(74) Representative: McLeish, Nicholas Alistair Maxwell
(86) International application number: PCT/US2000/032761
(87) International publication number: WO 2002/043798

(56) References cited:
- EP-A- 0 784 991
- WO-A-98/58697
- WO-A-99/58183
- US-A- 5 308 324
- US-A- 5 792 055
- US-A- 6 019 736
- US-A- 6 142 975

## Description

### Field of the Invention:

This invention relates generally to vascular interventional medical devices, and more particularly concerns guide wires for use in a therapeutic system or for delivery of medical devices.

### Description of Related Art:

Conventional minimally invasive catheter based therapies typically require guidewires that are one to two meters long extending through a longitudinal lumen in the catheter, and that are torqueable and pushable at the proximal end, yet soft and flexible at the distal end. Many such guidewires are made of stainless steel or the like, and are ground to tapers which provide the desired bending properties along the guidewire. It is useful for such guidewires to be torqueable from the base of the guidewire for manipulation of the distal tip, which is typically bent, for guiding the distal tip through vascular passages. While such guidewires need to be torqueable, pushable and resilient, particularly at the proximal regions of the guidewire, they also need to be flexible, particularly at the distal regions of the guidewire.

One prior guidewire for use with a catheter includes a core wire formed from a nickel titanium alloy, with a tapered distal tip portion and a distal end cap, covered by a sheath of material such as polyurethane, polyethylene, nylon, silicone, polytetrafluoroethylene, cellulose, starch or gelatin. Another prior guidewire comprises a composite guidewire with a core of stainless steel or a nickel titanium alloy, a tapered distal region ending in a distal flexible coil and end cap, also having a major portion of the guidewire covered by a thin layer of polymeric material, such as polysulfones, polyfluorocarbons, polyolefins, polyesters, polyamides, polyurethanes, blends and copolymers such as polyether block amides.

EP-A-0784991 discloses a guidewire with a relatively stiff proximal segment and a distal segment with a tapered segment between the proximal and distal segments. A coiled wire spring surrounds at least a portion of the distal segment. A sleeve is secured around a proximal portion of the guidewire and may be sized to fit snugly over the outer surface of the proximal segment.

However, there remains a need for a guidewire with enhanced proximal stiffness, with a stiff, high modulus reinforcement, allowing for greater manipulation of the guidewire by the physician, along with greater distal tip flexibility with radiopacity. The present invention meets these needs.

### SUMMARY OF THE INVENTION

Briefly, and in general terms, the present invention provides an improved composite guidewire with a proximal high modulus reinforcement member for promoting greater proximal stiffness, with a tapered distal region and distal radiopaque coil providing greater distal tip flexibility with radiopacity. The composite structure thus advantageously provides for a composite guidewire with greater resilience, a transition in stiffness, and tip flexibility. The proximal stiffer, high modulus member, covering or reinforcing a nickel alloy core, can be formed of high modulus metals such as stainless steel, titanium, and the like, allowing for greater manipulation of the guidewire by the physician, while the distal coil has enhanced durability, being formed from a composite strand of a nickel titanium alloy and platinum.

According to the present invention there is provided a composite guidewire as claimed in the appended claims.

In a presently preferred embodiment, the core is a nickel titanium alloy rod, although the core may alternatively be formed of one or more elongated strands of nickel titanium alloy, or an elongated tube. In another presently preferred aspect, the proximal reinforcement member is formed as an elongated ground stainless steel hypo tube, although the reinforcement member may alternatively be formed of an elongated tube made of titanium, or a nickel titanium alloy. In another presently preferred aspect, the proximal reinforcement member is formed with a distal tapered portion, to provide for a transition in stiffness of the guidewire. In a presently preferred embodiment, the heat shrinkable coating is formed from an elongated tube of polytetrafluoroethylene (PTFE), although the heat shrinkable coating may also be selected from other heat shrinkable materials such as polyethylene, for example. In another presently preferred aspect of the invention, the distal primary coil is formed from one or more nickel titanium alloy strands or wires, and in another presently preferred aspect the distal primary coil is formed from one or more platinum wires, or a combination of one or more nickel titanium alloy strands and one or more platinum wires. In one currently preferred embodiment, the distal tip is formed of platinum, and is bonded to the distal end of the core such as by welding, or soldering, or the like, although the distal tip may also be formed of other materials such as a tantalum filled epoxy adhesively bonded to the distal end of the core.

These and other aspects and advantages of the invention will become apparent from the following detailed description and the accompanying drawings, which illustrate by way of example the features of the invention.

### BRIEF DESCRIPTION OF THE DRAWINGS

Figure 1 is a longitudinal sectional schematic diagram of the composite guidewire of the invention;
Fig. 2 is a transverse sectional view taken along line 2-2 of Figure 1;
Fig. 3 is a transverse sectional view similar to Fig. 2 illustrating a first alternate preferred embodiment;
Fig. 4 is a transverse sectional view similar to Fig. 2 illustrating a second alternate preferred embodiment;
Fig. 5 is a transverse sectional view taken along line 5-5 of Figure 1;
Fig. 6 is a transverse sectional view taken along line 6-6 of Figure 1;
Fig. 7 is a transverse sectional view taken along line 7-7 of Figure 1;
Fig. 8 is a transverse sectional view taken along line 8-8 of Figure 1;

### DETAILED DESCRIPTION OF THE PREFERRED EMBODIMENTS

Guidewires used for vascular therapeutic intervention typically need to be torqueable, pushable and resilient over a proximal region of the guidewire, and flexible, over the distal region of the guidewire. While tapered guidewires can provide a range of proximal stiffness and torqueability to distal flexibility, enhancement of the proximal stiffness of such guidewires can give a physician manipulating the guidewire better control over the distal positioning of the guidewire.

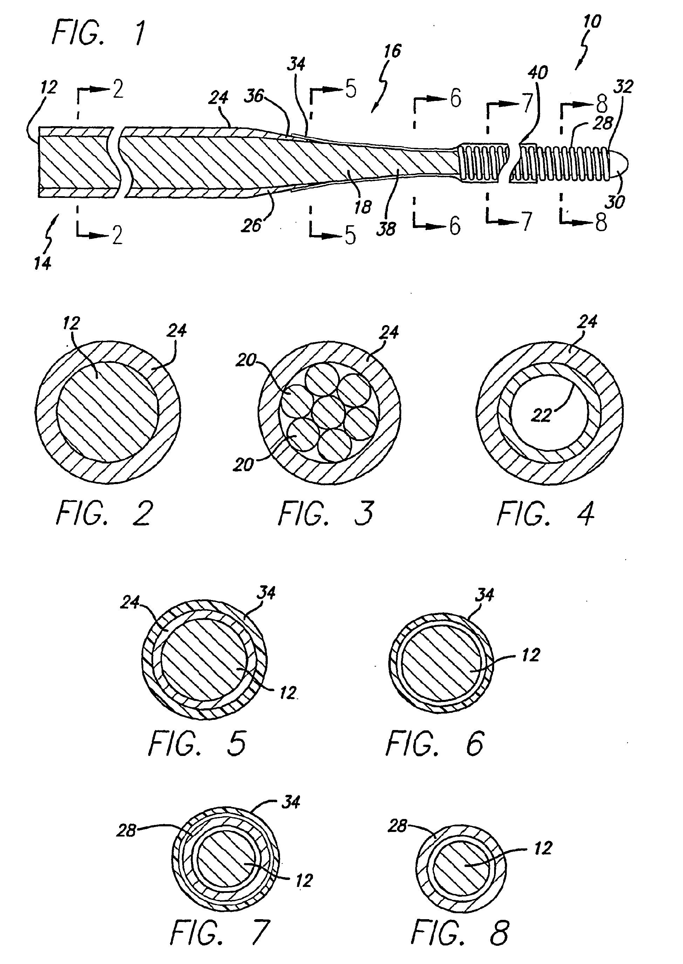

As is illustrated in the drawings, the invention is embodied in a composite guidewire 10 illustrated in Figure 1, having a central elongated, flexible core 12 formed from a nickel titanium alloy such as Nitinol, having a proximal region 14 and a distal region 16. The distal region of the core includes a tapered portion 18, to provide for a gradual transition to increased flexibility in the distal region of the guidewire. The core is preferably formed as an elongated rod, as illustrated in Fig. 2, although the core may also be formed of a one or more strands 20 of a nickel titanium alloy such as Nitinol as shown in Fig. 3. The one or more strands may be helically wound, or may run longitudinally parallel along the length of the guidewire. Alternatively, the core may also be formed from an elongated tube 22 such as a hypo tube made of a nickel titanium alloy such as Nitinol, as shown in Fig. 4.

A proximal reinforcement tube 24 is preferably disposed over the proximal region of the core, with the reinforcement tube having a tapered distal portion 26, to provide for a transition in stiffness of the guidewire. The proximal reinforcement member is currently preferably formed from an elongated ground stainless steel hypo rube, although the reinforcement member may alternatively be formed of an elongated tube made of titanium, or a nickel titanium alloy such as Nitinol.

A primary coil 28 is preferably bonded over the tapered distal region of the core, such as by welding, solder, or by adhesive such as cyanoacrylate. The primary coil is currently preferably formed from one or more nickel titanium alloy strands or wires as described above, one or more platinum wires to provide radiopacity to the primary coil, or a combination of one or more nickel titanium alloy strands and one or more platinum wires. A distal tip 30 is secured to the distal end 32 of the core and to the distal end 32 of the primary coil. In one currently preferred embodiment, the distal tip is formed of platinum, and is bonded to the distal end of the core such as by welding, or soldering, or the like, although the distal tip may also be formed of other materials such as a tantalum filled epoxy adhesively bonded to the distal end of the core and to the distal end of the primary coil.

An outer coating of a heat shrinkable polymeric material 34, such as an elongated tube of polytetrafluoroethylene (PTFE), is also disposed over at least a distal portion of the reinforcement member 36, an intermediate portion 38 of the core, and at least a portion 40 of the distal primary coil. The heat shrinkable coating may also be selected from other similar suitable heat shrinkable materials such as polyethylene, for example.

It will be apparent from the foregoing that while particular forms of the invention have been illustrated and described, various modifications can be made without departing from the scope of the invention. Accordingly, it is not intended that the invention be limited, except as by the appended claims.

## Claims

1. A composite guidewire (10), including an elongated, flexible core (12) formed from a nickel titanium alloy, said core (12) having a proximal region (14), a distal region (16), and an intermediate tapered region (18, 38) between said proximal and distal regions; a reinforcement tube (24) disposed over said proximal region (14) of said core (12); a primary coil (28) disposed over said distal region (16) of said core (12); and a distal tip (30) secured to the distal end (32) of said core (12), said composite guidewire (10) **characterized by**:
said reinforcement tube (24) including a distal tapered portion (26) extending over a portion of said intermediate tapered region (18) of said elongated, flexible core (12), and a coating (34) of a heat shrinkable material disposed over at least a portion of said distal tapered portion (26, 36) of said reinforcement tube (24), said intermediate tapered region (18, 38) of said core (12), and at least a portion (40) of said primary coil (28).

2. The composite guidewire (10) of Claim 1, wherein said reinforcement tube (24) comprises an elongated ground stainless steel hypo tube.

3. The composite guidewire (10) of Claim 1, wherein said reinforcement tube (24) comprises titanium.

4. The composite guidewire (10) of Claim 1, wherein said reinforcement tube (24) comprises a nickel titanium alloy.

5. The composite guidewire (10) of Claim 1, wherein said coating (34) of a heat shrinkable material comprises an elongated tube of polytetrafluoroethylene.

6. The composite guidewire (10) of Claim 1, wherein said primary coil (28) comprises at least one nickel titanium alloy strand.

7. The composite guidewire (10) of Claim 1, wherein said primary coil (28) comprises at least one platinum wire.

8. The composite guidewire (10) of Claim 6, wherein said primary coil (28) further comprises at least one platinum wire.

9. The composite guidewire (10) of Claim 1, wherein said distal tip (30) comprises platinum.

10. The composite guidewire (10) of Claim 1, wherein said distal tip (30) comprises tantalum filled epoxy.

11. The composite guidewire (10) of Claim 1, wherein said elongated, flexible core (12) is formed from a plurality of strands, and said plurality of strands are helically wound.

12. The composite guidewire (10) of Claim 1, wherein said elongated, flexible core (12) is formed from a plurality of strands, and said plurality of strands run longitudinally parallel along the length of the guidewire (10).

## Patentansprüche

1. Führungsdraht (10) aus Verbundstoff, der einen länglichen, biegsamen Kern (12) einschließt, welcher aus einer Nickel-Titan-Legierung gebildet ist, wobei der Kern (12) einen proximalen Bereich (14), einen distalen Bereich (16) und zwischen den proximalen und distalen Bereichen einen verjüngten Zwischenbereich (18, 38) aufweist; ein über dem proximalen Bereich (14) des Kerns (12) vorgesehenes Verstärkungsrohr (24); eine über dem distalen Bereich (16) des Kerns (12) vorgesehene Hauptwicklung (28); und eine am distalen Ende (32) des Kerns (12) befestigte, distale Spitze (30), wobei der Führungsdraht (10) aus Verbundstoff **dadurch gekennzeichnet ist, dass**
das Verstärkungsrohr (24) einen distalen verjüngten Abschnitt (26) einschließt, der sich über einen Abschnitt des verjüngten Zwischenabschnitts (18) des länglichen, biegsamen Kerns (12) erstreckt, und durch einen Überzug (34) aus einem Wärmeschrumpfmaterial, der über zumindest einem Abschnitt des distalen verjüngten Abschnitts (26, 36) des Verstärkungsrohrs (24), dem verjüngten Zwischenbereich (18, 38) des Kerns (12) und zumindest einem Abschnitt (40) der Hauptwicklung (28) vorgesehen ist.

2. Führungsdraht (10) aus Verbundstoff gemäß Anspruch 1, bei welchem das Verstärkungsrohr (24) ein längliches, geschliffenes Hyporohr aus rostfreiem Stahl umfasst.

3. Führungsdraht (10) aus Verbundstoff gemäß Anspruch 1, bei welchem das Verstärkungsrohr (24) Titan umfasst.

4. Führungsdraht (10) aus Verbundstoff gemäß Anspruch 1, bei welchem das Verstärkungsrohr (24) eine Nickel-Titan-Legierung umfasst.

5. Führungsdraht (10) aus Verbundstoff gemäß Anspruch 1, bei welchem der Überzug (34) aus einem Wärmeschrumpfmaterial ein längliches Rohr aus Polytetrafluorethylen umfasst.

6. Führungsdraht (10) aus Verbundstoff gemäß Anspruch 1, bei welchem die Hauptwicklung (28) zumindest einen Faden aus einer Nickel-Titan-Legierung umfasst.

7. Führungsdraht (10) aus Verbundstoff gemäß Anspruch 1, bei welchem die Hauptwicklung (28) zumindest einen Platindraht umfasst.

8. Führungsdraht (10) aus Verbundstoff gemäß Anspruch 6, bei welchem die Hauptwicklung (28) weiter zumindest einen Platindraht umfasst.

9. Führungsdraht (10) aus Verbundstoff gemäß Anspruch 1, bei welchem die distale Spitze (30) Platin umfasst.

10. Führungsdraht (10) aus Verbundstoff gemäß Anspruch 1, bei welchem die distale Spitze (30) mit Tantal gefülltes Epoxy umfasst.

11. Führungsdraht (10) aus Verbundstoff gemäß Anspruch 1, bei welchem der längliche, biegsame Kern (12) aus einer Mehrzahl von Fäden gebildet ist und die Mehrzahl von Fäden schraubenförmig gewickelt ist.

12. Führungsdraht (10) aus Verbundstoff gemäß Anspruch 1, bei welchem der längliche, biegsame Kern (12) aus einer Mehrzahl von Fäden gebildet ist und die Mehrzahl von Fäden in Längsrichtung parallel entlang der Länge des Führungsdrahts (10) verläuft.

## Revendications

1. Fil-guide composite (10), comprenant une partie centrale flexible, allongée (12) formée à partir d'un alliage de titane et nickel, ladite partie centrale (12) ayant une zone proximale (14), une zone distale (16) et une zone conique intermédiaire (18, 38) entre lesdites zones proximale et distale ; un tube de renfort (24) disposé sur ladite zone proximale (14) de ladite partie centrale (12) ; une bobine primaire (28) disposée sur ladite zone distale (16) de ladite partie centrale (12) ; et une pointe distale (30) fixée à l'extrémité distale (32) de ladite partie centrale (12), ledit fil-guide composite (10) étant **caractérisé par** :
ledit tube de renfort (24) comprenant une portion conique distale (26) s'étendant sur une portion de ladite zone conique intermédiaire (18) de ladite partie centrale flexible allongée (12), et un revêtement (34) d'un matériau thermo-rétrécissable disposé sur au moins une partie de ladite portion conique distale (26, 36) dudit tube de renfort (24), ladite zone conique intermédiaire (18, 38) de ladite partie centrale (12) et au moins une portion (40) de ladite bobine primaire (28).

2. Fil-guide composite (10) selon la revendication 1, dans lequel ledit tube de renfort (24) comprend un hypo-tube en acier inoxydable rectifié allongé.

3. Fil-guide composite (10) selon la revendication 1, dans lequel ledit tube de renfort (24) comprend du titane.

4. Fil-guide composite (10) selon la revendication 1, dans lequel ledit tube de renfort (24) comprend un alliage de titane et nickel.

5. Fil-guide composite (10) selon la revendication 1, dans lequel ledit revêtement (34) en un matériau thermo-rétrécissable comprend un tube allongé en polytétrafluoroéthylène.

6. Fil-guide composite (10) selon la revendication 1, dans lequel ladite bobine primaire (28) comprend au moins un toron d'alliage de titane et nickel.

7. Fil-guide composite (10) selon la revendication 1, dans lequel ladite bobine primaire (28) comprend au moins un fil de platine.

8. Fil-guide composite (10) selon la revendication 6, dans lequel ladite bobine primaire (28) comprend en outre au moins un fil de platine.

9. Fil-guide composite (10) selon la revendication 1, dans lequel ladite pointe distale (30) comprend du platine.

10. Fil-guide composite (10) selon la revendication 1, dans lequel ladite pointe distale (30) comprend une résine époxy chargée de tantale.

11. Fil-guide composite (10) selon la revendication 1, dans lequel ladite partie centrale flexible, allongée (12) est formée d'une pluralité de torons, et ladite pluralité de torons est enroulée de manière hélicoïdale.

12. Fil-guide composite (10) selon la revendication 1, dans lequel ladite partie centrale flexible allongée (12) est formée d'une pluralité de brins, et ladite pluralité de brins s'étend longitudinalement de manière parallèle sur la longueur du fil-guide (10).
